# FASCICULE DE BREVET EUROPEEN

(11) **EP 3 236 888 B1**
(45) Date de publication et mention de la délivrance du brevet: **26.09.2018**
(21) Numéro de dépôt: 15823655.4
(22) Date de dépôt: 17.12.2015
(51) Int. Cl.: A61F 5/01, A61F 13/06

(54) **DISPOSITIF DE PROTECTION DU CAPITON PLANTAIRE EN PARTICULIER LORS D'UNE ACTIVITE SPORTIVE**
VORRICHTUNG ZUM SCHUTZ DER SOHLENPOLSTERUNG, INSBESONDERE BEI EINER SPORTLICHE BETÄTIGUNG
DEVICE FOR PROTECTING THE PLANTAR PADDING, IN PARTICULAR DURING A SPORTING ACTIVITY

(30) Priorité: 24.12.2014 FR 1463309
(43) Date de publication de la demande: 01.11.2017
(73) Titulaire: Millet Innovation, 26270 Loriol sur Drome (FR)
(72) Inventeur: MILLET, Damien, 26000 Valence (FR); GRANGE, Odile, 26400 Allex (FR); FONTAINE, Thierry, 26740 Marsanne (FR)
(74) Mandataire: de Roquemaurel, Bruno
(86) Numéro de dépôt international: PCT/FR2015/053565
(87) Numéro de publication internationale: WO 2016/102824

(56) Documents cités:
- FR-A1- 2 712 487
- FR-A1- 2 892 298
- US-A- 5 497 789
- US-A1- 2010 249 686
- US-B1- 6 318 373

## Description

La présente invention concerne un dispositif de protection du pied notamment pour répartir la charge sur l'avant de la plante du pied.

Le pied possède une couche absorbante de chocs appelée "capiton plantaire", capable de supporter jusqu'à huit fois le poids du corps. Le capiton plantaire permet de répartir la "charge" mécanique imposée par le poids du corps sur les métatarsiens. Or, de nombreux patients souffrent d'échauffement, de durillons ou de douleurs liés à une usure inévitable avec l'âge du capiton plantaire naturel qui ne joue alors plus aussi efficacement son rôle de répartition de charge. En vieillissant, le capiton plantaire a tendance à s'amincir et durcir. Le pied peut également être atteint d'affections cutanées (irritations, fendillements, verrues plantaires...). Par ailleurs, certaines pratiques sportives conduisent à solliciter le capiton plantaire d'une manière intense pouvant entrainer des traumatismes, ainsi que sa dégradation à long terme.

Pour soulager ou prévenir ces algies et/ou affections, il est connu de placer sous les têtes métatarsiennes et/ou sous les articulations métatarso-phalangiennes un coussinet dans un matériau viscoélastique choisi pour répartir les charges, afin d'éviter la formation d'hyperkératoses locales, généralement appelées durillon. Pour réaliser un tel coussinet, il est également connu d'utiliser une plaquette à base de gel polymère tel que du gel de silicone ou à base d'hydrogel pour assurer une fonction de substitut au capiton plantaire. Ainsi, une telle plaquette, de quelques centimètres carrés, peut être réalisée à partir d'une composition de gel silicone de type PDMS (polydiméthylsiloxane), par exemple commercialisée par la demanderesse sous l'appellation Epithélium 26®. Le brevet FR 2 712 487 décrit un tel gel de silicone ayant des propriétés se rapprochant de celles du capiton plantaire utilisé pour la prévention de pathologies d'hyper appuis apparaissant essentiellement sur ou sous les pieds.

La présente invention vise particulièrement à protéger le capiton plantaire des sportifs. Ceux-ci ont un capiton plantaire sain, mais la pratique sportive conduit à le solliciter de manière intense, ce qui justifie la prévision d'une protection permettant de prévenir ses traumatismes immédiats et sa dégradation à long terme. Or les contraintes liées à l'effet recherché d'un tel coussinet sur la peau s'avèrent souvent contradictoires avec les contraintes liées au maintien du coussinet sur la zone à protéger, voire même avec les bonnes conditions d'exercice du sport concerné.

Pour assurer une fonction de répartition de charge, il est connu d'utiliser du gel de silicone relativement dur. Toutefois, les gels de silicone de ce type sont très peu adhésifs, et en tout état de cause, insuffisamment adhésifs pour tenir en place naturellement sur la zone à protéger. Par ailleurs, pour assurer une répartition de charge suffisante, le coussinet doit présenter une certaine épaisseur de plusieurs millimètres, ce qui s'oppose également au maintien du coussinet sur la zone à protéger. En effet, plus les bords du coussinet sont épais plus ils risquent d'être accrochés, entrainant l'arrachement du coussinet. Il existe des gels de silicone plus adhésifs, mais plus mous, ce qui les rend moins résistants. Ils présentent donc une durée d'utilisation réduite. Il est en effet souhaitable que le coussinet puisse supporter une longue durée d'utilisation et donc plusieurs lavages. Quoi qu'il en soit, ils ne se prêtent pas bien à une production industrielle car ils ne peuvent pas être correctement manipulés dans un processus industriel qui inclut l'usage de tissus et des opérations d'assemblage par couture.

Ne pouvant exploiter l'adhésivité naturelle d'un gel de silicone sur la peau, il est connu d'assurer la tenue du coussinet par des moyens de maintien en tissu élastique. Toutefois, de tels moyens de maintien peuvent former à l'intérieur de la chaussure une couche de matériau qui s'ajoute à celle formée par la chaussette, risquant d'altérer le confort du pied dans la chaussure. Par ailleurs, la viscoélasticité du coussinet et son épaisseur ne doivent pas induire d'effets pervers sur la posture de l'utilisateur, en statique comme en dynamique. Pour les sportifs, une instabilité posturale peut avoir des effets en chaine sur les muscles et les articulations, peut altérer la précision de leurs gestes et occasionner des blessures.

En outre, durant un usage sportif, de brusques changements de direction et des changements d'appui répétitifs peuvent entrainer un déplacement du coussinet par rapport au pied et notamment une rotation autour du pied.

La demanderesse a développé et commercialise un coussinet adapté aux séniors ayant un capiton plantaire usé (plus mince et plus dur). Ce coussinet forme une surépaisseur sous le pied supérieure à 3 mm pour une épaisseur de matériau viscoélastique de 2,2 mm. Ce matériau présente une dureté évaluée par un indice de pénétration (mesuré conformément à la norme NF T77-104 - avril 1986), compris entre 81 et 95. Il s'avère que ce coussinet remplit parfaitement sa fonction de compensation de l'usure du capiton plantaire sans perturber la posture de l'utilisateur lors de la marche ordinaire. En revanche, ce coussinet n'est pas adapté pour un usage sportif. En effet, le mode de maintien du coussinet sur le pied est insuffisant dans le cadre d'un usage sportif intense. En outre, son épaisseur excessive tend à perturber la posture de l'utilisateur et le confort du pied dans une chaussure de sport. Il convient ici de rappeler que l'équilibre de la position debout résulte de l'intégration en permanente de données venant de trois systèmes récepteurs, à savoir : le système visuel, le système proprioceptif et le système vestibulaire.

Le système visuel (position par rapport à l'environnement) et le système vestibulaire (détection des accélérations rotatoires et linéaires de la tête dans l'espace par un organe de l'oreille) ne devraient pas être perturbés par la présence d'un coussinet sous le pied. Il en va différemment pour le système proprioceptif, car la perception du sol par les mécanorécepteurs cutanés et profonds est atténuée par l'interposition du coussinet. Or la prise en compte des signaux venant de ces capteurs conduit des réactions musculaires permettant de faire disparaître la sensation consciente ou non consciente d'un déséquilibre. Ces réactions musculaires peuvent être à l'origine de pathologies temporaires comme les crampes, ou plus longues comme les tendinites dans toute la cinématique du corps.

Il peut donc être souhaitable de prévoir un coussinet assurant une fonction de répartition de la charge sans entrainer d'instabilité posturale de l'utilisateur, associé à des moyens de maintien aptes à maintenir le coussinet en place sur le pied, y compris durant une pratique sportive sollicitant les pieds de manière intense. Il peut donc être également souhaitable que l'ensemble du coussinet et des moyens de maintien ne soient pas trop volumineux afin d'être compatible avec le port de chaussures sans introduire de gêne. Il peut être également souhaitable que le coussinet ne puisse pas provoquer des blessures, notamment sur les orteils et des réactions de la peau. Il peut être également souhaitable que l'ensemble du coussinet et des moyens de maintien puisse être utilisé pendant plusieurs mois et puisse être fabriqué facilement.

Des modes de réalisation concernent un dispositif de protection du capiton plantaire d'un pied, comprenant : un coussinet comportant un matériau viscoélastique, prévu pour être maintenu sur la peau de la région plantaire, et couvrant l'ensemble des têtes métatarsiennes et des articulations métatarso-phalangiennes du pied, et une bande élastique fixée au coussinet le long de deux bords transversaux opposés du coussinet et par une patte prévue pour passer entre le gros orteil et l'orteil adjacent du pied, la bande présentant une largeur apte à couvrir entièrement des protubérances latérales interne et externe du pied, formées par les articulations métatarso-phalangiennes du petit orteil et du gros orteil, une partie de la bande destinée à venir en contact avec le dessus du pied, comportant sur ses deux faces des éléments adhérents prévus pour empêcher le dispositif de protection de glisser sur la peau lorsqu'il est disposé sur le pied et sur l'intérieur d'une chaussette enveloppant le pied.

Selon un mode de réalisation, les éléments adhérents présentent la forme de picots ou d'une structure quadrillée ou en nid d'abeille, et sont réalisés en un gel polymère.

Selon un mode de réalisation, les éléments adhérents formés sur l'une des deux faces de la bande présentent la forme d'une structure quadrillée ou en nid d'abeille.

Selon un mode de réalisation, les éléments adhérents forment chacun une surépaisseur au-dessus de la surface du tissu formant la bande, comprise entre 0,4 et 0,6 mm.

Selon un mode de réalisation, le coussinet forme une poche dans laquelle est logée une plaquette en un matériau viscoélastique.

Selon un mode de réalisation, la plaquette est réalisée en gel polymère présentant une dureté correspondant à un indice de pénétration compris entre 30 et 80 mesuré à l'aide d'un pénétromètre, et une épaisseur comprise entre 1 et 1,8 mm.

Selon un mode de réalisation, la plaquette est réalisée en gel polymère présentant une dureté correspondant à un indice de pénétration compris entre 70 et 100 mesuré à l'aide d'un pénétromètre, et présente une épaisseur comprise entre 0,3 et 1 mm.

Selon un mode de réalisation, le coussinet comprend une pièce de tissu externe présentant une épaisseur comprise entre 0,5 et 1 mm et une pièce de tissu interne destinée à venir en contact avec la peau du pied, présentant une épaisseur comprise entre 0,2 et 0,6 mm.

Selon un mode de réalisation, la pièce de tissu externe, la bande et la patte appartiennent à une même pièce de tissu élastique.

Selon un mode de réalisation, la bande présente le long des bords latéraux du pied une largeur comprise entre 35 et 45 mm.

Selon un mode de réalisation, la bande présente une épaisseur comprise entre 0,5 et 1 mm.

Selon un mode de réalisation, le coussinet présente une forme s'inscrivant dans un rectangle ayant une longueur minimum comprise entre 83 et 105 mm et une largeur minimum comprise entre 56 et 68 mm.

Selon un mode de réalisation, la bande est fixée au coussinet pour maintenir le coussinet sur le pied de manière à ce qu'un bord proximal du coussinet forme avec un axe longitudinal du pied un angle compris entre 70 et 80°.

Des exemples de réalisation de l'invention seront décrits dans ce qui suit, à titre non limitatif en relation avec les figures jointes parmi lesquelles :
les figures 1A et 1B sont respectivement des vues de dessous et de dessus schématiques d'un dispositif de protection du pied, selon un mode de réalisation,
la figure 2 représente schématiquement le dispositif de protection en coupe transversale suivant la ligne AA' indiquée sur la figure 1A,
la figure 3 représente schématiquement la forme d'un coussinet du dispositif de protection,
les figures 4 et 5 sont respectivement des vues schématiques de dessus et de dessous d'un pied équipé du dispositif de protection,
les figures 6A et 6B représentent schématiquement, respectivement une face extérieure et une face intérieure du dispositif de protection, selon un mode de réalisation,
la figure 7 représente sous la forme d'un graphe des plages de valeurs d'épaisseur et de dureté prévues pour le coussinet,
la figure 8 représente schématiquement une face intérieure ou extérieure du dispositif de protection, selon un autre mode de réalisation,
la figure 9 est une vue en coupe transversale schématique du dispositif de protection à une étape intermédiaire de fabrication.

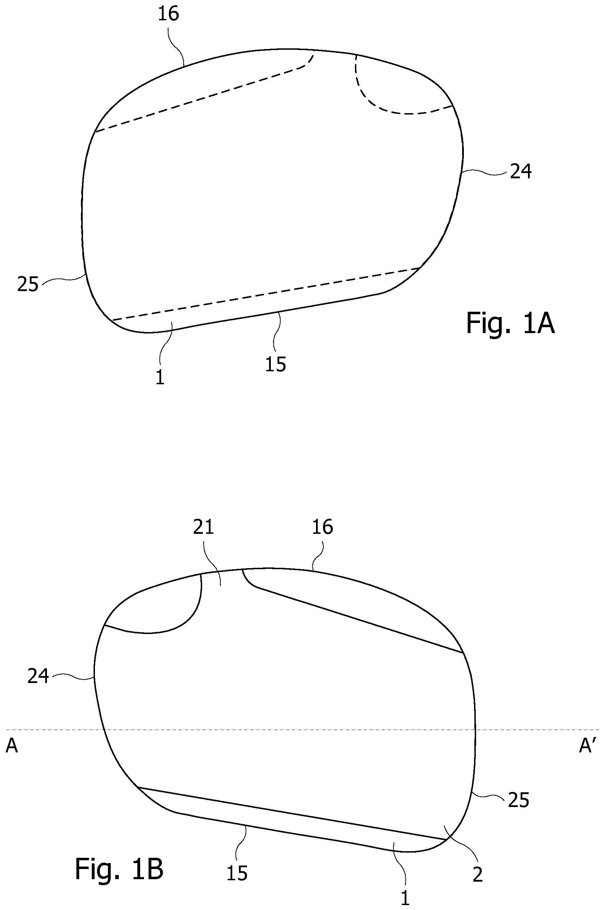

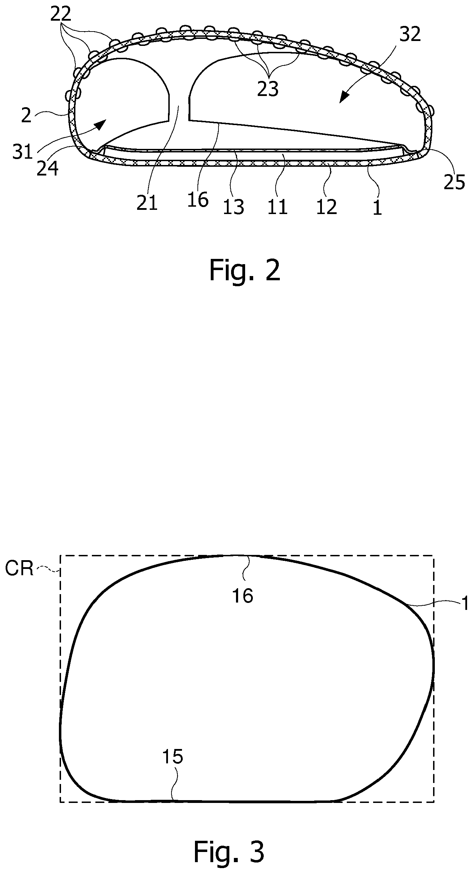

Les figures 1A, 1B et 2 représentent un dispositif de protection du pied, selon un mode de réalisation. Le dispositif comprend un coussinet 1 et une bande élastique 2 fixée au coussinet 1 le long de deux bords transversaux opposés 24, 25 du coussinet. L'ensemble du coussinet 1 et de la bande 2 forme ainsi un manchon à deux ouvertures opposées, ajusté de manière à serrer légèrement l'avant du pied. La bande 2 est également fixée au coussinet 1 par une patte 21 solidaire de la bande 2 et fixée sur un bord longitudinal distal 16 du coussinet. La patte 21 délimite ainsi deux ouvertures 31, 32, l'ouverture 31 étant prévue pour le passage du gros orteil du pied et l'ouverture 32 étant prévue pour le passage des autres orteils.

Selon un mode de réalisation, la bande 2 est réalisée dans un tissu élastique, par exemple un tissu indémaillable, et présente une épaisseur comprise entre 0,5 et 1 mm, par exemple de l'ordre de 0,6 mm.

Le coussinet 1 comprend une plaquette 11 en un matériau viscoélastique adapté à assurer une fonction de répartition de charge. Selon un mode de réalisation, la plaquette est logée dans une poche formée par une pièce de tissu externe 12 assemblée avec une pièce de tissu interne 13. La forme et les dimensions de la poche peuvent être ajustées précisément à celles de la plaquette 11. La plaquette 11 peut être collée sur toute sa surface sur l'une des deux pièces de tissu 12, 13, par exemple la pièce de tissu externe 12. Les pièces de tissu externe 12 et interne 13 peuvent être élastiques et présenter une épaisseur comprise entre 0,5 et 0,8 mm, par exemple de l'ordre de 0,6 mm. Selon un mode de réalisation, la pièce de tissu interne 13 présente une épaisseur plus faible, par exemple comprise entre 0,2 et 0,6 mm, par exemple de l'ordre de 0,2 mm.

Selon un mode de réalisation, la plaquette 11 est réalisée en un gel polymère viscoélastique, par exemple en un gel de silicone. Ainsi, la plaquette 11 peut par exemple être réalisée en PDMS (polydiméthylsiloxane) présentant une dureté relativement élevée, correspondant à un indice de pénétration par exemple compris entre 30 et 80 (mesuré à l'aide d'un pénétromètre - cf. norme NF T 77-104, avril 1986). Ces valeurs d'indice de pénétration sont fournies pour établir des comparaisons entre différents matériaux viscoélastiques, sachant que la norme NF T 77-104 préconise de limiter les mesures de cet indice à une plage allant de 85 à 400. A titre de comparaison, les plaquettes utilisées dans les coussinets pour les séniors présentent un indice de pénétration, mesuré dans les mêmes conditions de 81 à 95. La plaquette 11 peut présenter une épaisseur comprise entre 1 et 1,8 mm, par exemple de l'ordre de 1,5 mm. De cette manière l'épaisseur totale du dispositif de protection sous le pied peut atteindre au minimum 1,7 mm. Dans l'exemple ci-dessus, la proportion de l'épaisseur de matériau viscoélastique par rapport à l'épaisseur totale du dispositif sous le pied peut atteindre 71% (1,5 / 2,1). Dans ces conditions d'épaisseur et de dureté de la plaquette 11 et d'épaisseur de son enveloppe en tissu, la plaquette 11 est en mesure d'assurer une protection efficace en présence d'un capiton plantaire sain, sans perturber la stabilité posturale de l'utilisateur tout en occupant un espace minimum dans le chaussant.

La figure 3 représente le coussinet 1. Le coussinet 1 s'inscrit dans un rectangle CR, dont un grand côté coïncide avec plus des deux tiers d'un bord proximal 15 du coussinet. Il est à noter que le rectangle CR est le plus petit rectangle dans lequel le coussinet 1 peut être inscrit. Selon les tailles des pieds d'adulte, le coussinet s'inscrit dans un rectangle CR présentant une longueur comprise entre 83 et 105 mm et une largeur comprise entre 56 et 68 mm. Le rectangle CR peut ainsi présenter des dimensions minimum de 83 x 56 mm et des dimensions maximum de 105 x 68 mm.

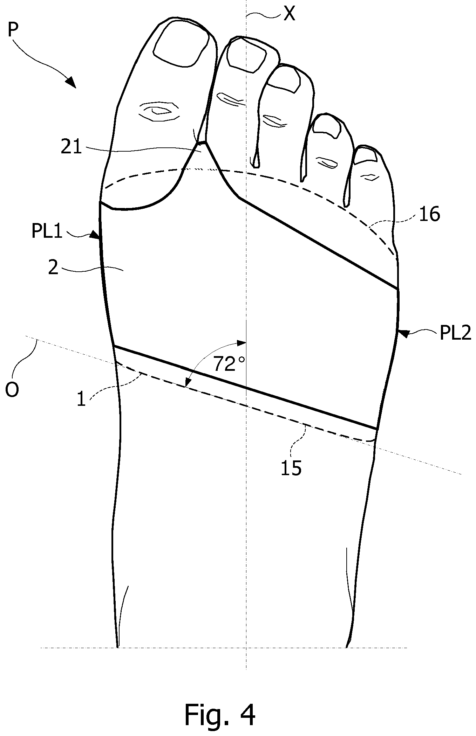

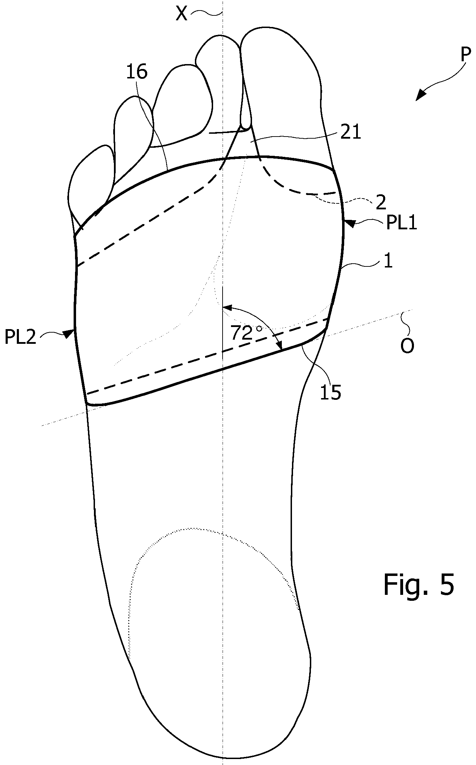

Comme illustré sur les figures 4 et 5 montrant le dispositif de protection disposé sur un pied, la forme et les dimensions du coussinet 1 (et donc de la plaquette 11) sont prévues pour couvrir la totalité des têtes métatarsiennes et des articulations métatarso-phalangiennes du pied, et le cas échéant, une partie des deux bords latéraux du pied. Le coussinet 1 est prévu pour être disposé sur le pied de manière à ce que son bord distal 16 coïncide sensiblement avec une ligne passant par la base des orteils dans le cas d'un pied de type égyptien. Dans cette position, le bord proximal 15 du coussinet 1 qui s'étend suivant un axe O (correspondant à la direction des grands côtés du rectangle CR), forme un angle avec l'axe longitudinal X du pied (passant par le centre du talon et le milieu de l'extrémité du deuxième orteil) compris entre 70 et 80° selon la morphologie du pied. Dans l'exemple des figures 4 et 5, cet angle est de 72°.

La bande 2 est configurée pour couvrir entièrement les protubérances latérales interne PL1 et externe PL2 du pied, formées par les articulations entre le métatarsien et la première phalange du gros orteil et du petit orteil, respectivement. Il apparaît que cette disposition contribue au maintien en place du coussinet 1 sur le pied en empêchant notamment les glissements du dispositif de protection suivant l'axe du pied. La bande 2 présente sensiblement une même largeur (à 15% près), comprise entre 35 et 45 mm, sur les côtés latéraux du pied.

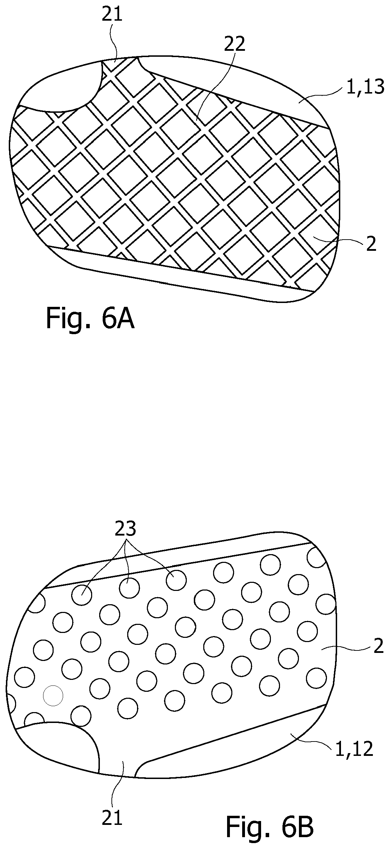

La figure 6A représente le dispositif de protection, selon un mode de réalisation, dans une position montrant la face externe de la bande 2. La figure 6B représente le dispositif de protection selon un mode de réalisation, dans une configuration retournée pour montrer la face interne de la bande 2, destinée à venir en contact avec la peau du pied. Dans des modes de réalisation illustrés par les figures 2, 6A et 6B, la bande 2 est recouverte des deux côtés par des éléments adhérents 22, 23 en surépaisseur, prévus pour empêcher le dispositif de glisser sur le pied ou autour de ce dernier, en particulier lorsque ce dernier est serré dans une chaussure. Le serrage exercé par la bande élastique 2, conjugué à la forme de cette bande et à l'adhérence et à la forme des éléments adhérents 22, 23 permet d'assurer le maintien du dispositif de protection sur le pied, y compris lors de pratiques sportives impliquant de fortes accélérations et des changements brusques de direction. A cet effet, il n'est pas nécessaire que la force de serrage exercée par la bande 2 autour du pied soit excessive au point de provoquer une gêne de l'utilisateur, même si le dispositif de protection est porté durant plusieurs heures.

Les éléments adhérents 22, 23 peuvent être formés en gel de silicone, et fixés sur le tissu par enduction, en déposant sur le tissu formant la bande 2, le gel de silicone sous une forme suffisamment liquide (avant réticulation complète) pour pénétrer dans le tissu afin d'obtenir une tenue mécanique suffisante sans apport de colle.

Sur la figure 6A, les éléments adhérents externes 22 formés sur la face externe de la bande 2 présentent une structure quadrillée formée de bandes parallèles régulièrement espacées s'entrecroisant avec d'autres bandes parallèles régulièrement espacées. Les éléments adhérents externes 22 peuvent recouvrir tout ou partie de la patte 21 et des parties destinées à être situées sur les bords latéraux du pied. Une telle structure quadrillée ou en nid d'abeille présente l'avantage de donner une certaine tenue ou une certaine rigidité au tissu formant la bande 2. Il s'avère que cette caractéristique contribue au maintien en place du dispositif sur le pied.

Sur la figure 6B, les éléments adhérents internes 23 formés sur la face interne de la bande 2 présentent la forme de picots en forme de calotte sphérique, uniformément répartis sur la face interne de la bande 2, y compris sur les parties de la bande 2 destinées à venir en contact avec les parties latérales du pied. Pour ne pas gêner l'utilisateur, la face interne de la patte 21 peut ne pas être recouverte d'éléments adhérents.

Les éléments adhérents 22, 23 forment chacun une surépaisseur au-dessus de la surface du tissu formant la bande 2, comprise entre 0,4 et 0,6 mm. Ainsi, l'ensemble de la bande 2 et des éléments adhérents 22, 23 présente une épaisseur comprise entre 1,5 et 2 mm, par exemple de l'ordre de 1,6 mm. Cette épaisseur additionnée à celle du coussinet 1, de l'ordre de 4,2 mm reste faible. A ce sujet, il convient de noter que les éléments 23 pénètrent partiellement dans la peau, et qu'en présence d'une chaussette, les éléments 22 pénètrent partiellement dans l'épaisseur de celle-ci, ce qui contribue également au maintien du dispositif de protection sur le pied. Dans ces conditions, l'encombrement du dispositif de protection reste suffisamment faible pour être compatible avec le port de chaussettes et chaussures sans affecter le confort de l'utilisateur.

Il est à noter que même si les éléments adhérents externes 22 pénètrent entièrement dans l'épaisseur d'une chaussette, ils génèrent des pressions localisées s'exerçant sur l'intérieur de la chaussure, ces pressions contribuant au maintien du dispositif de protection sur le pied. A noter également que la chaussette reste généralement en place dans la chaussure, sans être soumise à des déplacements en rotation autour du pied. Elle assure donc également une certaine retenue de la bande 2 par l'intermédiaire des éléments adhérents externes 22.

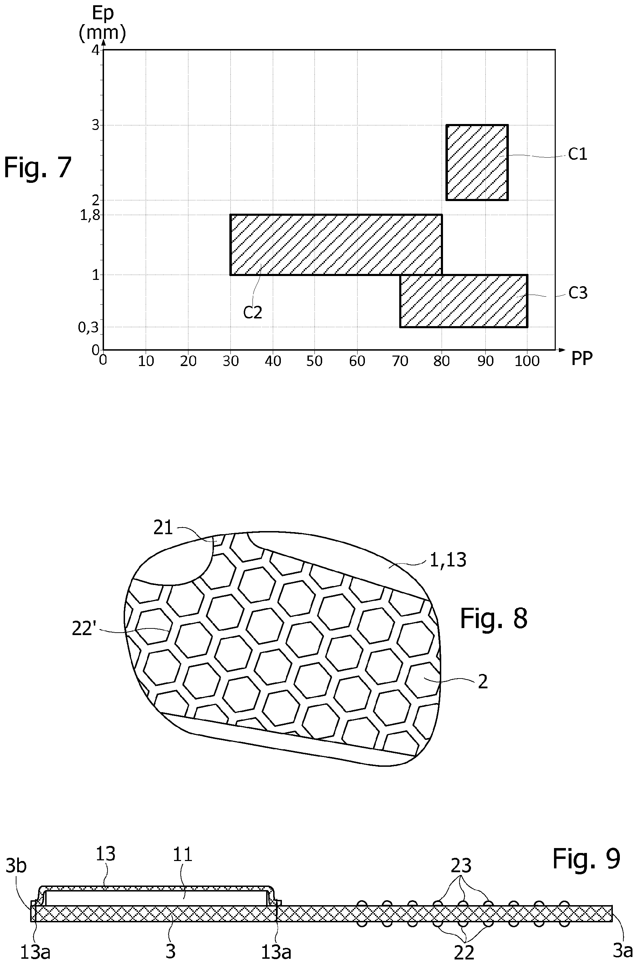

La figure 7 représente un graphe illustrant des plages de valeurs envisagées pour l'épaisseur Ep et la dureté PP de la plaquette 11, la dureté étant exprimée sous la forme de valeurs d'indice de pénétration mesurées à l'aide d'un pénétromètre. La figure 7 représente des plages de valeurs C1, C2, C3. La plage C1 correspond aux valeurs d'épaisseur et de dureté de la plaquette selon l'art antérieur, utilisée pour les séniors ayant un capiton plantaire usé. La plage C1 est définie entre les limites de dureté PP de 81 et 95 et entre les limites d'épaisseur EP de 2 et 3 mm. La plage C2 correspond à une plaquette utilisée pour assurer une fonction de répartition de charge (c'est-à-dire de forces verticales), sans conséquence notable sur la stabilité posturale. La plage C2 est définie entre les limites de dureté PP de 30 et 80, et entre les limites d'épaisseur Ep de 1 et 1,8 mm.

Selon un mode de réalisation, la plaquette 11 peut également présenter des valeurs de dureté et d'épaisseur appartenant à la plage C3. La plage C3 est définie pour assurer à la fois une fonction de répartition de charge et une fonction de protection contre les échauffements du capiton plantaire. De tels échauffements apparaissent notamment lors du port de chaussures faiblement ou non amortissantes, et/ou lors de la pratique de sports imposant de fortes accélérations dirigées parallèlement au sol, c'est-à-dire aussi bien suivant l'axe du pied (lors de démarrages et freinages), que suivant un axe perpendiculaire, par exemple en cas de changements d'appui de pied brutaux. La plage C3 est définie entre les limites de dureté PP de 70 et 100 et entre les limites d'épaisseur EP de 0,3 et 1 mm. La plaquette 11 dans la plage C3 est plus molle que dans la plage C2 pour absorber des forces de cisaillement apparaissant sous l'effet de forces parallèles au sol. La plaquette 11 dans la plage C3 est également plus fine que dans la plage C2 pour limiter l'instabilité posturale susceptible d'apparaître du fait que la plaquette est plus molle que dans la plage C2. Des mesures ont permis de confirmer que l'instabilité posturale générée par un coussinet renfermant une plaquette 11 appartenant à la plage C3 n'est pas plus importante qu'avec une plaquette appartenant à la plage C2. En revanche, cette instabilité se trouve plus importante avec une plaquette appartenant à la plage C1. Le protocole utilisé pour mesurer l'impact du coussinet sur la stabilité posturale comporte des phases de mesure d'une force médio latérale (parallèle au sol et perpendiculaire à l'axe X du pied), dont une phase de mesure sans coussinet et des phases de mesures avec des coussinets sous les pieds, pendant lesquelles des expérimentateurs courent pendant une période de plusieurs minutes. Les résultats obtenus durant ces phases de mesures permettent d'évaluer par comparaison l'importance des compensations nécessitées par l'usage des coussinets. Ainsi, plus la force médio latérale mesurée est faible, plus grandes auront été les compensations engagées par l'expérimentateur pour retrouver le même équilibre. A noter que les mesures ont été effectuées pieds nus, car le port de chaussures influence également la stabilité posturale en relation avec la bonne adéquation de la taille et du serrage des chaussures.

Aux faibles épaisseurs inférieures à 1,5 mm, la plaquette 11 peut être formée par enduction de la pièce de tissu 12 ou 13 (ou 3), le cas échéant, par plusieurs étapes d'enduction successives pour atteindre l'épaisseur souhaitée.

Il est à noter que des éléments adhérents tels que les éléments adhérents 22 ou 23, peuvent également être prévus sur la face externe de la pièce de tissu externe 12. Par ailleurs, les éléments adhérents formés sur la face interne de la bande peuvent être en forme de structure quadrillée ou en nid d'abeille.

Les éléments adhérents peuvent présenter d'autres formes. Ainsi la figure 8 représente une face interne ou externe du dispositif de protection sur laquelle sont formés des éléments adhérents 22' présentant une structure en nid d'abeille. A noter que les mailles des structures quadrillée ou en nid d'abeille des éléments adhérents 22, 22' ne présentent pas nécessairement des côtés de mêmes longueurs, ces structures pouvant être étirées dans une direction.

Comme illustré par la figure 9 représentant le dispositif de protection à une étape intermédiaire de fabrication, celui-ci peut être fabriqué en réalisant la pièce de tissu externe 12, la patte 21 et la bande 2 décrites précédemment, en une pièce de tissu unique 3. La plaquette 11 et les éléments adhérents 22, 23 peuvent ensuite être formés ou fixés sur la pièce de tissu 3. La pièce de tissu interne 13 peut être ensuite fixée sur la pièce de tissu 3 en recouvrant la plaquette 11 de manière à former une poche, par exemple par une couture 13a formée le long du bord de la plaquette. La pièce de tissu 3 peut ensuite être refermée sur elle-même de manière à former un manchon, par exemple en réalisant une couture bord à bord de bords opposés 3a, 3b. L'extrémité libre de la patte 21 peut être fixée de la même manière sur un bord opposé de la pièce de tissu 3. Dans l'exemple de la figure 9, ces coutures peuvent être réalisées en partie sur la couture 13a.

Il n'est pas nécessaire que la couture 13a s'étende sur l'ensemble du contour de la plaquette 11. Il importe simplement que la pièce de tissu 13 ne puisse pas s'écarter et ainsi permettre à la plaquette de venir en contact de la peau du pied. Il n'est pas non plus nécessaire que la bande 2 et la pièce de tissu externe 12 soient formées par une même pièce de tissu. Dans ce cas, la bande 2 peut être assemblée avec la pièce 12 par trois coutures, à savoir deux coutures opposées et une couture pour fixer l'extrémité libre de la patte 21 formée dans la bande 2.

Grâce à ces dispositions, l'ensemble du dispositif est formé dans des matériaux (tissus, gel de silicone) adaptés à être placés en contact avec la peau pendant de longues durées. En outre, ces matériaux présentent l'avantage d'être lavables plusieurs fois, permettant ainsi au dispositif de protection d'être utilisé durant plusieurs mois. Il est à noter également que le dispositif de protection ne présente pas de grandes surfaces en matériau étanche à l'eau. En effet, la plaquette 11 n'est pas en contact direct avec la peau, la pièce de tissu 13 étant disposée entre la plaquette et la peau, et les éléments adhérents 22, 23 présentent de faibles surfaces. Il en résulte que le dispositif de protection n'empêche pas l'évacuation de la transpiration qui pourrait notamment réduire l'adhérence du dispositif sur la peau.

Il apparaîtra clairement à l'homme de l'art que la présente invention est susceptible de diverses variantes de réalisation. En particulier, l'invention n'est pas limitée à l'usage de gel de silicone en tant que matériau viscoélastique pour la plaquette 11 et les éléments adhérents 22, 23. Ainsi, ces éléments peuvent être réalisés dans d'autres matériaux tels que le néoprène, les polymères d'uréthane, etc.

## Revendications

1. Dispositif de protection du capiton plantaire d'un pied, comprenant :
un coussinet (1) comportant un matériau viscoélastique, prévu pour être maintenu sur la peau de la région plantaire, et couvrant l'ensemble des têtes métatarsiennes et des articulations métatarso-phalangiennes du pied, et
une bande élastique (2) fixée au coussinet le long de deux bords transversaux opposés (24, 25) du coussinet et par une patte (21) prévue pour passer entre le gros orteil et l'orteil adjacent du pied, la bande présentant une largeur apte à couvrir entièrement des protubérances latérales interne et externe du pied, formées par les articulations métatarso-phalangiennes du petit orteil et du gros orteil, une partie de la bande destinée à venir en contact avec le dessus du pied, comportant sur ses deux faces des éléments adhérents (22, 23) prévus pour empêcher le dispositif de protection de glisser sur la peau lorsqu'il est disposé sur le pied et sur l'intérieur d'une chaussette enveloppant le pied.

2. Dispositif selon la revendication 1, dans lequel les éléments adhérents (22, 23) présentent la forme de picots ou d'une structure quadrillée ou en nid d'abeille, et sont réalisés en un gel polymère.

3. Dispositif selon la revendication 1 ou 2, dans lequel les éléments adhérents (22, 23) formés sur l'une des deux faces de la bande (2) présentent la forme d'une structure quadrillée ou en nid d'abeille.

4. Dispositif selon l'une des revendications 1 à 3, dans lequel les éléments adhérents (22, 23) forment chacun une surépaisseur au-dessus de la surface du tissu formant la bande (2), comprise entre 0,4 et 0,6 mm.

5. Dispositif selon l'une des revendications 1 à 4, dans lequel le coussinet (1) forme une poche dans laquelle est logée une plaquette (11) en un matériau viscoélastique.

6. Dispositif selon la revendication 5, dans lequel la plaquette (11) est réalisée en gel polymère présentant une dureté correspondant à un indice de pénétration compris entre 30 et 80 mesuré à l'aide d'un pénétromètre, et une épaisseur comprise entre 1 et 1,8 mm.

7. Dispositif selon la revendication 5, dans lequel la plaquette (11) est réalisée en gel polymère présentant une dureté correspondant à un indice de pénétration compris entre 70 et 100 mesuré à l'aide d'un pénétromètre, et présente une épaisseur comprise entre 0,3 et 1 mm.

8. Dispositif selon la revendication 5 à 7, dans lequel le coussinet (1) comprend une pièce de tissu externe (12) présentant une épaisseur comprise entre 0,5 et 1 mm et une pièce de tissu interne (13) destinée à venir en contact avec la peau du pied, présentant une épaisseur comprise entre 0,2 et 0,6 mm.

9. Dispositif selon la revendication 8, dans lequel la pièce de tissu externe (12), la bande (2) et la patte (21) appartiennent à une même pièce de tissu élastique.

10. Dispositif selon l'une des revendications 1 à 9, dans lequel la bande (2) présente le long des bords latéraux du pied une largeur comprise entre 35 et 45 mm.

11. Dispositif selon l'une des revendications 1 à 10, dans lequel la bande (2) présente une épaisseur comprise entre 0,5 et 1 mm.

12. Dispositif selon l'une des revendications 1 à 11, dans lequel le coussinet (1) présente une forme s'inscrivant dans un rectangle (CR) ayant une longueur minimum comprise entre 83 et 105 mm et une largeur minimum comprise entre 56 et 68 mm.

13. Dispositif selon l'une des revendications 1 à 12, dans lequel la bande (2) est fixée au coussinet (1) pour maintenir le coussinet sur le pied de manière à ce qu'un bord proximal (15) du coussinet forme avec un axe longitudinal (X) du pied un angle compris entre 70 et 80°.

## Patentansprüche

1. Vorrichtung zum Schutz der Sohlenpolsterung eines Fußes, umfassend:
ein Kissen (1), aufweisend ein viskoelastisches Material, das vorgesehen ist, auf der Haut der Sohlenregion gehalten zu werden, und die Gesamtheit der Mittelfußknochenköpfe und der Mittelfuß-Zehenglied-Gelenke des Fußes bedeckt, und
ein an dem Kissen entlang von zwei gegenüberliegenden transversalen Rändern (24, 25) des Kissens und mittels einer Lasche (21), die vorgesehen ist, zwischen dem großen Zeh und dem benachbarten Zeh des Fußes zu verlaufen, befestigter elastischer Streifen (2), wobei der Streifen eine Breite aufweist, die imstande ist, innere und äußere seitliche Vorsprünge des Fußes, die von den Mittelfuß-Zehenglied-Gelenken des kleinen Zehs und des großen Zehs gebildet sind, vollständig zu bedecken, wobei ein Teil des Streifens, der bestimmt ist, mit der Oberseite des Fußes in Kontakt zu kommen, auf seinen zwei Seiten Haftelemente (22, 23) aufweist, die vorgesehen sind, zu verhindern, dass die Schutzvorrichtung auf der Haut rutscht, wenn sie auf dem Fuß und auf dem Inneren einer den Fuß umhüllenden Socke angeordnet ist.

2. Vorrichtung nach Anspruch 1, wobei die Haftelemente (22, 23) die Form von Noppen oder einer Karo- oder Wabenstruktur aufweisen und aus einem Polymergel hergestellt sind.

3. Vorrichtung nach Anspruch 1 oder 2, wobei die auf der einen der zwei Seiten des Streifens (2) gebildeten Haftelemente (22, 23) eine Karo- oder Wabenstruktur aufweisen.

4. Vorrichtung nach einem der Ansprüche 1 bis 3, wobei die Haftelemente (22, 23) jeweils eine Verdickung auf der Oberfläche des Stoffs, welche den Streifen (2) bildet, zwischen 0,4 und 0,6 mm inklusive bilden.

5. Vorrichtung nach einem der Ansprüche 1 bis 4, wobei das Kissen (1) eine Tasche bildet, in welcher eine Platte (11) aus einem viskoelastischen Material untergebracht ist.

6. Vorrichtung nach Anspruch 5, wobei die Platte (11) aus einem Polymergel hergestellt ist, das eine Härte, die einem Penetrationsindex zwischen 30 und 80 inklusive entspricht, gemessen mit Hilfe eines Penetrometers, und eine Stärke zwischen 1 und 1,8 mm inklusive aufweist.

7. Vorrichtung nach Anspruch 5, wobei die Platte (11) aus einem Polymergel hergestellt ist, das eine Härte aufweist, die einem Penetrationsindex zwischen 70 und 100 inklusive entspricht, gemessen mit Hilfe eines Penetrometers, und eine Stärke zwischen 0,3 und 1 mm inklusive aufweist.

8. Vorrichtung nach Anspruch 5 bis 7, wobei das Kissen (1) ein äußeres Stoffteil (12) umfasst, das eine Stärke zwischen 0,5 und 1 mm inklusive aufweist, und ein inneres Stoffteil (13), das bestimmt ist, mit der Haut des Fußes in Kontakt zu kommen, das eine Stärke zwischen 0,2 und 0,6 mm inklusive aufweist.

9. Vorrichtung nach Anspruch 8, wobei das äußere Stoffteil (12), der Streifen (2) und die Lasche (21) zu demselben elastischen Stoffteil gehören.

10. Vorrichtung nach einem der Ansprüche 1 bis 9, wobei der Streifen (2) entlang der seitlichen Ränder des Fußes eine Breite zwischen 35 und 45 mm inklusive aufweist.

11. Vorrichtung nach einem der Ansprüche 1 bis 10, wobei der Streifen (2) eine Stärke zwischen 0,5 und 1 mm aufweist.

12. Vorrichtung nach einem der Ansprüche 1 bis 11, wobei das Kissen (1) eine Form aufweist, die in ein Rechteck (CR) mit einer Mindestlänge zwischen 83 und 105 mm inklusive und einer Mindestbreite zwischen 56 und 68 mm inklusive passt.

13. Vorrichtung nach einem der Ansprüche 1 bis 12, wobei der Streifen (2) an dem Kissen (1) befestigt ist, um das Kissen derart an dem Fuß zu halten, dass ein proximaler Rand (15) des Kissens mit einer Längsachse (X) des Fußes einen Winkel zwischen 70 und 80° inklusive bildet.

## Claims

1. A device for protecting the plantar padding of a foot, comprising:
a cushion (1) including a viscoelastic material, configured to be held against the skin of the plantar region, and covering the metatarsal heads and the metatarsophalangeal joints of the foot, and
an elastic band (2) attached to the cushion along two opposing transverse edges (24, 25), and by a tab (21) configured to pass between the big toe and the adjacent toe of the foot, the band having a width configured to entirely cover internal and external side bulges formed by the metatarsophalangeal joints of the small and big toes, wherein a portion of the band configured for contacting the top of the foot includes tacky elements (22, 23) on both sides thereof, thereby preventing the protective device from sliding on the skin when it is placed on the foot and inside a sock surrounding the foot.

2. The device of claim 1, wherein the tacky elements (22, 23) are in the form of studs, or have a grid or honeycomb structure, and are made of polymer gel.

3. The device of claim 1 or 2, wherein the tacky elements (22, 23) of one side of the band (2) have a grid or honeycomb structure.

4. The device of any of claims 1 to 3, wherein the tacky elements (22, 23) protrude from the surface of the fabric forming the band (2) by 0.4 to 0.6 mm.

5. The device of any of claims 1 to 4, wherein the cushion (1) includes a pocket holding a pad (11) made of viscoelastic material.

6. The device of claim 5, wherein the pad (11) is made of polymer gel having a hardness corresponding to a penetration index between 30 and 80 measured with a penetrometer, and has a thickness between 1 and 1.8 mm.

7. The device of claim 5, wherein the pad (11) is made of polymer gel having a hardness corresponding to a penetration index between 70 and 100 measured with a penetrometer, and has a thickness between 0.3 and 1 mm.

8. The device of any of claims 5 to 7, wherein the cushion (1) comprises an external fabric piece (12) having a thickness between 0.5 and 1 mm, and an internal fabric piece (13) configured to contact the skin of the foot, having a thickness between 0.2 and 0.6 mm.

9. The device of claim 8, wherein the external fabric piece (12), the band (2) and the tab (21) are integral with a same piece of elastic fabric.

10. The device of any of claims 1 to 9, wherein the band (2) has, along the sides of the foot, a width between 35 and 45 mm.

11. The device of any of claims 1 to 10, wherein the band (2) has a thickness between 0.5 and 1 mm.

12. The device of any of claims 1 to 10, wherein the cushion (1) has a shape included within a rectangle (CR) having a length of at least 83 to 105 mm, and a width of at least 56 to 68 mm.

13. The device of any of claims 1 to 12, wherein the band (2) is attached to the cushion (1) for maintaining the cushion on the foot such that a proximal edge (15) of the cushion forms an angle between 70 and 80° with a longitudinal axis (X) of the foot.
